# EUROPÄISCHE PATENTSCHRIFT

(11) **EP 2 419 152 B1**
(45) Veröffentlichungstag und Bekanntmachung des Hinweises auf die Patenterteilung: **10.12.2014**
(21) Anmeldenummer: 10714207.7
(22) Anmeldetag: 16.04.2010
(51) Int. Cl.: A61L 27/24, A61L 27/38

(54) **IMPLANTAT UND THERAPEUTISCHE ZUSAMMENSETZUNG ZUR BEHANDLUNG VON SCHÄDEN UND/ODER ERKRANKUNGEN IM BEREICH DES MENSCHLICHEN UND/ODER TIERISCHEN STÜTZ- UND BEWEGUNGSAPPARATES**
IMPLANT AND THERAPEUTIC COMPOSITION FOR TREATING DAMAGE AND/OR DISEASES RELATING TO THE HUMAN AND/OR ANIMAL MUSCULOSKELETAL SYSTEM
IMPLANT ET COMPOSITION THÉRAPEUTIQUE POUR LE TRAITEMENT DE TROUBLES ET/OU DE MALADIES DANS LE DOMAINE DE L'APPAREIL MUSCULO-SQUELETTIQUE ET MOTEUR HUMAIN ET/OU ANIMAL

(30) Priorität: 17.04.2009 DE 102009018640
(43) Veröffentlichungstag der Anmeldung: 22.02.2012
(73) Patentinhaber: TETEC Tissue Engineering Technologies AG, 72770 Reutlingen (DE)
(72) Erfinder: MOLLENHAUER, Jürgen, 72764 Reutlingen (DE)
(74) Vertreter: Patentanwälte Ruff, Wilhelm, Beier, Dauster & Partner
(86) Internationale Anmeldenummer: PCT/EP2010/002329
(87) Internationale Veröffentlichungsnummer: WO 2010/118874

(56) Entgegenhaltungen:
- EP-A1- 1 254 670
- WO-A2-2007/035778
- US-A1- 2003 152 556
- US-A1- 2005 186 673
- US-B1- 6 444 222

## Beschreibung

Die vorliegende Erfindung betrifft ein Implantat und eine therapeutische Zusammensetzung, welche sich vor allem zur Behandlung von Schäden und/oder Erkrankungen im Bereich des menschlichen und/oder tierischen Stütz- und/oder Bewegungsapparates eignen, sowie ein Herstellungsverfahren für das Implantat und die therapeutischen Zusammensetzung.

Artikulärer Knorpel ist lediglich in begrenztem Ausmaß fähig, Gelenkflächenschäden oder Schäden zwischen den Wirbelkörpern der Wirbelsäule zu reparieren. Eine Zusammensetzung auf Basis von Kollagen, die sich prinzipiell auch zur Reparatur von Knorpelgewebe eignet, ist aus der EP 0 747 067 A2 bekannt.

Ein weiteres grundlegendes Konzept zur chirurgischen Behandlung von Knorpelschäden sieht den Einsatz von Knorpelimplantaten auf der Basis von in-vitro hergestellten Knorpelzellen vor. Die Knorpelimplantate sollten dabei möglichst autologe Zellen aufweisen, um das Risiko von Abstoßungsreaktionen bei den betroffenen Patienten möglichst gering zu halten.

Da mit Hilfe von Biopsien gewöhnlich nur wenige autologe Zellen bereitgestellt werden können, werden die isolierten Knorpelzellen in der Regel mehrfachen Passagen unterworfen. Problematisch ist hierbei jedoch, dass mit zunehmender Anzahl der Passagen ein fortschreitender Verlust des differenzierten zellulären Phänotyps einhergeht. Dies bedeutet, dass sich die isolierten Knorpelzellen hinsichtlich ihrer Eigenschaften immer weiter von ihrem natürlichen Original entfernen, je häufiger sie einem technischen Vermehrungszyklus unterworfen werden. Darüber hinaus steigt mit zunehmender Anzahl an Passagen das Risiko für eine Mutation und damit allgemein für eine Tumorgenese.

Ein weiteres Problem bei der in vitro-Züchtung von Knorpelzellen in Zellkulturschalen, -flaschen oder dergleichen besteht darin, dass diese Zellen gewöhnlich nicht von einer extrazellularen Matrix umgeben sind, die etwa von den Knorpelzellen produzierte und sezernierte Stoffe zurückhalten könnte. Vielmehr verteilen sich die zellulär ausgeschiedenen Stoffe, beispielsweise lösliche Prokollagene, nicht ausgereifte Proteoglykane, Glykoprotein-Untereinheiten, Gewebehormone, Wachstumsfaktoren sowie spezifische extrazellulare Proteasen, diffus in der künstlichen Umgebung. Um diesen Stoff bzw. Materialverlust zu kompensieren, müssen die vorstehend genannten und andere Stoffe von den Knorpelzellen ständig nachproduziert und erneut ausgeschieden werden. Dies bedeutet, dass die Knorpelzellen eine permanent erhöhte Stoffwechselrate aufweisen, was für die Knorpelzellen wiederum erhöhte Stressbedingungen bedeutet. Dies kann zu einer Beeinträchtigung von Knorpelimplantaten führen, die auf solchen Knorpelzellen basieren.

Die US 2005/0186673 A1 offenbart eine im Wesentlichen aus Kollagen des Typs II gebildete Matrix, welche zur Förderung des Zellwachstums mit einer Nukleinsäure beladen ist.

Aus der WO 2007/035778 A2 ist eine Trägermatrix für Zellen bekannt, welche unter anderem aus Kollagen des Typs I, II und/oder IV gebildet sein kann.

Gegenstand der US 6,444,222 B1 ist eine verstärkte Matrix, welche Kollagen sowie ein gerüstbildendes Protein, vorzugsweise Elastin, aufweist.

Die US 2003/0152556 A1 beschreibt ein Knorpelimplantat mit Chondrozyten oder mesenchymalen Stammzellen sowie einem Substrat, welches Kollagen des Typs I in Form von α-helikalen Monomeren enthält.

Die EP 1 254 670 A1 betrifft eine Matrix zur Herstellung oder Regeneration von Knorpelgewebe, welche aus einem Netzwerk von modifziertem Kollagen des Typs II besteht.

Aufgabe der vorliegenden Erfindung ist es daher, ein Implantat bereitzustellen, welches aus dem Stand der Technik bekannte Nachteile vermeidet und insbesondere eine gegenüber herkömmlichen Knorpelimplantaten verbesserte und insbesondere beschleunigte Knorpelneubildung, d.h. Chondrogenese, ermöglicht.

Diese Aufgabe wird erfindungsgemäß gelöst durch ein Implantat mit den Merkmalen des unabhängigen Anspruchs 1. Bevorzugte Ausführungsformen des Implantats sind Gegenstand der abhängigen Ansprüche 2 bis 10. Eine therapeutische Zusammensetzung ist Gegenstand des unabhängigen Anspruchs 11. Ein Verfahren zur Herstellung eines Implantats ist Gegenstand des Anspruchs 12. Ein weiterer Gegenstand der Erfindung betrifft ein Verfahren zur Herstellung einer therapeutischen Zusammensetzung mit den Merkmalen des unabhängigen Anspruchs 13. Schließlich betrifft die Erfindung auch Verwendungen gemäß den Ansprüchen 14 und 15.

Bei dem erfindungsgemäßen Implantat handelt es sich um ein Implantat, umfassend ein Trägermaterial, Knorpelzellen und/oder Vorläuferzellen davon und einen knorpelspezifischen Kollagen-Typ. Das Implantat kann grundsätzlich sowohl in der Humanmedizin als auch in der Veterinärmedizin eingesetzt werden. Die Verwendung in der Humanmedizin, insbesondere auf dem Gebiet der regenerativen Medizin, ist bevorzugt. So eignet sich das Implantat insbesondere zur Behandlung von Schäden und/oder Erkrankungen im Bereich des menschlichen und/oder tierischen Stütz- und/oder Bewegungsapparates.

Überraschenderweise konnte festgestellt werden, dass Knorpelzellen bzw. deren Vorläuferzellen knorpelspezifisches Kollagen des Typs VI, welches ihnen in vitro angeboten wird, "einfangen" und zum Aufbau einer Art extrazellularen Matrix verwenden. Durch den Aufbau einer extrazellularen Matrix um die Knorpelzellen herum können die von den Zellen sezernierten Stoffe besser zurückgehalten werden, wodurch der einleitend beschriebene Stoffverlust der Zellen vermindert wird. Dadurch wird die zelluläre Metabolismusrate gedrosselt, wodurch sich insgesamt der Stress für die Knorpelzellen verringert. Der geringere zelluläre Stress sowie der Aufbau einer extrazellularen Matrix bewirken insgesamt verbesserte knorpelinduzierende, d.h. chondrogene, Eigenschaften bei erfindungsgemäßen Implantaten gegenüber konventionellen Implantaten.

In einer bevorzugten Ausführungsform sind die Knorpelzellen und/oder deren Vorläuferzellen autologen und/oder allogenen, vorzugsweise autologen, Ursprungs. Die Knorpelzellen und/oder deren Vorläuferzellen können grundsätzlich tierischen Ursprungs sein und/oder aus geeigneten Zelllinien stammen. Die Knorpelzellen und/oder deren Vorläuferzellen können grundsätzlich xenogenen Ursprungs sein. Beispielsweise kann es sich bei den erfindungsgemäß vorgesehenen Knorpelzellen und/oder deren Vorläuferzellen um Zellen von Schweinen, Pferden, Rindern, Dromedaren, Hunden und/oder Katzen handeln. Bevorzugt sind die Knorpelzellen und/oder deren Vorläuferzellen humanen Ursprungs. Besonders bevorzugt ist die Verwendung von autologen humanen Knorpelzellen und/oder entsprechenden Vorläuferzellen. Die Knorpelzellen und/oder deren Vorläuferzellen können mittels dem Fachmann geläufigen Methoden aus menschlichen oder tierischen Knorpelgeweben, insbesondere Gelenkknorpelgeweben und/oder Bandscheibengeweben, isoliert und gegebenenfalls kultiviert werden.

Die Knorpelzellen sind in einer weiteren Ausführungsform aus der Gruppe bestehend aus Chondrozyten, Chondroblasten und Mischungen davon ausgewählt. Als Chondroblasten ("Knorpelbildner") werden allgemein die Vorläuferzellen der Chondrozyten bezeichnet. Sie stammen von mesenchymalen Stammzellen ab und stellen die aktive Form der Knorpelzelle dar, da sie alle Komponenten der Knorpelmatrix synthetisieren können. Sobald sie diese Synthesefunktion eingestellt haben, differenzieren sie sich zu den Chondrozyten, den eigentlichen Knorpelzellen. Die Chondrozyten selbst sind kleiner als die Chondroblasten, kugelig geformt, besitzen einen rundlichen Zellkern und enthalten viel Wasser, Fett und Glykogen. Ihre Anzahl, Lage und Dichte ist für jede Knorpelart spezifisch. Die Chondrozyten können sich im unausgereiften Zustand noch teilen, was zum charakteristischen Auftreten von sogenannten isogenen Gruppen führen kann. Die Entstehung dieser isogenen Gruppen beruht auf der Teilung von Chondrozyten, welche bereits von einer Knorpelmatrix umgeben sind und deswegen nicht mehr auseinanderweichen können. Mit anderen Worten handelt es sich bei den isogenen Gruppen um Chondrozyten-Komplexe, bei denen jeder Komplex aus einem einzigen Chondrozyten entstanden ist. Sobald die Chondrozyten ausdifferenziert sind, verlieren sie ihre Fähigkeit zur Teilung. Bei den Vorläuferzellen im Sinne der vorliegenden Erfindung handelt es sich in der Regel um Stammzellen, bevorzugt um mesenchymale Stammzellen. Die Stammzellen können tierischen oder humanen Ursprungs sein, wobei humane Stammzellen bevorzugt sind.

Grundsätzlich können die Knorpelzellen zwei- oder mehrfach passagiert sein. Unter einer Passage versteht man einen technischen Vermehrungszyklus, um die Anzahl an Zellen, welche gewöhnlich nur in geringer Zahl aus einem Biopsiematerial gewonnen werden können, zu erhöhen. So kann es sich bei den Knorpelzellen um sekundäre, tertiäre, quartäre Zellen usw. handeln. Bevorzugt handelt es sich bei den Knorpelzellen jedoch um primäre Knorpelzellen. Primäre Knorpelzellen haben den Vorteil, dass sie den natürlichen Knorpelzellen hinsichtlich der morphologischen und biochemischen Eigenschaften sehr nahe kommen.

Die Knorpelzellen und/oder deren Vorläuferzellen sind in einer besonders bevorzugten Ausführungsform durch eine in vitro-Besiedelung oder in vitro-Kultivierung in das Implantat eingelagert.

Der knorpelspezifische Kollagen-Typ kann grundsätzlich rekombinanten oder mikrobiologischen Ursprungs sein. Bevorzugt ist der knorpelspezifische Kollagen-Typ tierischen, insbesondere bovinen, porcinen und/ oder equinen, und/oder humanen Ursprungs.

In einer weitergehenden Ausführungsform stammt der knorpelspezifische Kollagen-Typ aus einem biologischen, insbesondere menschlichen oder tierischen, Gewebe. Vorzugsweise stammt der knorpelspezifische Kollagen-Typ aus einem Gewebe, ausgewählt aus der Gruppe bestehend aus Augen-Hornhaut, Plazentagewebe, Aortagewebe, Synovialgewebe und Mischungen davon.

Der knorpelspezifische Kollagen-Typ ist Kollagen des Typs VI. Die Verwendung von wasserlöslichem Kollagen des Typs VI ist erfindungsgemäß besonders bevorzugt. Bei diesem Kollagen-Typ handelt es sich um den Hauptbestandteil der sogenannten perizellularen Matrix, welche die Knorpelzellen unmittelbar umgibt. Die perizellulare Matrix umschließt käfigförmig einen oder mehrere Knorpelzellen. Die käfigförmige Anordnung aus perizellularer Matrix und Knorpelzellen wird auch als Chondron oder Territorium bezeichnet. Die perizellulare Matrix ist damit von der eigentlichen extrazellularen Matrix noch einmal zu unterscheiden. In natürlichem Knorpelgewebe umgibt die extrazellulare Matrix die soeben erwähnten Chondrone und damit die perizellulare Matrix. Kollagen des Typs VI ist ein Heterotrimer, bestehend aus den Polypeptiden α1 (VI), α2 (VI) und α3 (VI). Die vorstehend genannten Polypeptide weisen an ihren Enden jeweils eine globuläre Domäne auf. Die globulären Domänen sind in der Regel über einen kurzen tripelhelikalen Abschnitt voneinander beabstandet. Dadurch ergibt sich insgesamt eine hantelförmige Struktur für monomeres Kollagen des Typs VI. Die Monomere können sich über eine seitliche Verbindung zu Tetrameren zusammenlagern. Die Tetramere wiederum können über ihre Enden zu perlschnurförmigen und filamentartigen Strukturen zusammengelagert sein.

Das Implantat kann in einer weiteren Ausführungsform neben dem knorpelspezifischen Kollagen-Typ auch einen knorpel-untypischen Kollagen-Typ aufweisen, beispielsweise aus der Gruppe bestehend aus Kollagen des Typs I, III und Kombinationen davon.

Bevorzugt weist das Implantat zusätzlich biologische Wirkstoffe auf. Die Wirkstoffe können aus der Gruppe bestehend aus extrazelluläre Proteasen, Antikörper, Rezeptor-Antagonisten, Rezeptor-Agonisten, Hormone, Wachstumsfaktoren, Differenzierungsfaktoren, Rekrutierungsfaktoren, Adhäsionsfaktoren, Antibiotika, antimikrobielle Verbindungen, entzündungshemmende Verbindungen, immunsuppressive Verbindungen und Kombinationen davon ausgewählt sein.

Als Rekrutierungskomponenten sind beispielsweise Chemotaktika bzw. Chemotaxine geeignet. Als Adhäsionsfaktoren können beispielsweise Verbindungen aus der Gruppe bestehend aus Cytotactin, Laminin, Fibronectin, Kollagen des Typs IV, V und VII, synthetische Peptide, welche Teilsequenzen verschiedener Adhäsine darstellen, Transmembran-Verbindungsproteine, wie beispielsweise Integrin, und Kombinationen davon verwendet werden.

Bei den Wachstumsfaktoren handelt es sich vorzugsweise um Chondrogene, d. h. das Knorpelwachstum induzierende, Wachstumsfaktoren. Geeignete Wachstumsfaktoren können aus der Gruppe bestehend aus TGFs (Transforming Growth Factors), BMPs (Bone Morphogenetic Proteins), MPSF (Morphogenetic Protein Stimulatory Factors), Heparin bindende Wachstumsfaktoren, Inhibine, wachstumsdifferenzierende Faktoren, Aktivine und Kombinationen davon ausgewählt sein. Bevorzugt sind die Wachstumsfaktoren aus der Gruppe bestehend aus TGF-β1, TGF-β2, TGF-β3, BMP-1, BMP-2, BMP-3, BMP-4, BMP-5, BMP-6, BMP-7, BMP-8, BMP-9, FGF (Fibroblast Growth Factor), EGF (Epidermal Growth Factor), PDGF (Platelet-Derived Growth Factor), IGF (Insuline-like Growth Factor), Inhibin A, Inhibin B, GDF-I (Growth Differentiating Factor I), Aktivin A, Aktivin B, Aktivin AB und Kombinationen davon ausgewählt. Die Verwendung von TGFs und/oder BMPs ist besonders bevorzugt. Die Wachstumsfaktoren können aus nativen oder natürlichen Quellen, beispielsweise aus Säugetierzellen, isoliert sein. Erfindungsgemäß ist es ebenfalls möglich, dass die Wachstumsfaktoren synthetisch, beispielsweise mit Hilfe von rekombinanten DNA-Techniken oder durch chemische Methoden, hergestellt sind.

Besonders bevorzugt weist das Implantat Chondrone und/oder chondronartige Vorstufen auf. Wie bereits beschrieben, handelt es sich hierbei um käfigförmige Strukturen, die von einer perizellularen Matrix zusammen mit einer oder mehreren Knorpelzellen gebildet werden.

Das Trägermaterial ist vorzugsweise als dreidimensionale Struktur oder dreidimensionales Netzwerk bzw. Matrix, insbesondere Proteinmatrix, ausgebildet. Das Trägermaterial kann vernetzt oder unvernetzt vorliegen. Bevorzugt ist das Trägermaterial vernetzt, insbesondere chemisch vernetzt. Als Vernetzungsreagenzien können Aldehyde, Dialdehyde, Diisocyanate oder Carbodiimide verwendet werden.

Das Trägermaterial umfasst bevorzugt ein Material, welches ausgewählt ist aus der Gruppe bestehend aus Proteine bzw. Salze davon, Polysaccharide bzw. Salze davon, Polyhydroxyalkanoate, Calciumphosphate, tierische Membranen, Komposite davon und Kombinationen davon. Als besonders vorteilhafte Proteine sind vor allem Faserproteine bzw. Salze davon, insbesondere extrazelluläre Proteine bzw. Salze davon, bevorzugt ausgewählt aus der Gruppe bestehend aus Kollagen bzw. Salze davon, Gelatine bzw. Salze davon, Elastin bzw. Salze davon, Retikulin bzw. Salze davon und Kombinationen davon, zu nennen. Bevorzugte Kollagene sind aus der Gruppe bestehend aus Kollagen des Typs I, II, III und Kombinationen davon ausgewählt. Die Verwendung von Kollagen des Typs II als Trägermaterial ist besonders bevorzugt, da es sich hierbei um den kollagenen Hauptbestandteil der extrazellularen Matrix von natürlichem Knorpelgewebe handelt. Die Polysaccharide können aus der Gruppe bestehend aus Cellulosederivate, Chitosan, Chitonsanderivate, Glykosaminoglykane, Salze davon und Kombinationen davon ausgewählt sein. Bei den Cellulosederivaten handelt es sich bevorzugt um Alkylcellulosen, Hydroxyalkylcellulosen, Carboxyalkylcellulosen, Salze davon und/oder Kombinationen davon. Beispiele für geeignete Cellulosederivate können demnach aus der Gruppe bestehend aus Methylcellulose, Hydroxymethylcellulose, Hydroxyethylcellulose, Hydroxyethyl-methylcellulose, Hydroxypropylcellulose, Hydroxypropylmethylcellulose, Hydroxybutylcellulose, Carboxymethylcellulose, Salze davon und Kombinationen davon ausgewählt werden. Geeignete Glykosaminoglykane können aus der Gruppe bestehend aus Hyaluronsäure, Heparin, Heparansulfat, Chondroitin-4-sulfat, Chondroitin-6-sulfat, Dermatansulfat, Keratansulfat, Salze davon und Kombinationen davon ausgewählt werden. Als mögliche Polyhydroxyalkanoate sind vor allem Polyglykolid, Polylaktid, Poly-3-hydroxybutyrat, Poly-4-hydroxybutyrat, Copolymere davon und Mischungen davon zu nennen. Bevorzugte Calciumphosphate können aus der Gruppe bestehend aus Fluorapatite, Hydroxyapatite, Tricalciumphosphat, Tetracalciumphosphat und Kombinationen davon ausgewählt werden. Bei den tierischen Membranen handelt es sich vorzugsweise um Pericard, insbesondere bovinen Ursprungs.

Gegebenenfalls kann das Trägermaterial auch Verstärkungsstoffe, insbesondere Verstärkungsfasern, vorzugsweise ausgewählt aus der Gruppe bestehend aus Polysaccharidfasern, Proteinfasern, Seide, Baumwollfasern, Polylaktidfasern, Gelatinefasern und Kombinationen davon, enthalten.

Das Trägermaterial ist in der Regel porös, vorzugsweise offenporös, ausgebildet. Vorteilhafter Weise weist das Trägermaterial eine interkonnektierende Porosität auf. Das Trägermaterial kann weiterhin Poren mit einer Porengröße zwischen 50 und 500 µm, insbesondere 50 und 350 µm, bevorzugt 100 und 200 µm, aufweisen.

Das Trägermaterial ist in einer weiteren Ausführungsform mehrschichtig aufgebaut. Vorzugsweise weist das Trägermaterial zumindest eine erste Schicht und zumindest eine zweite Schicht auf. Die zumindest erste Schicht und die zumindest zweite Schicht sind in der Regel entlang einer gemeinsamen Grenzfläche miteinander verbunden. Die Verbindung entlang der gemeinsamen Grenzfläche kann auf chemischen und/oder physikalischen Bindungen beruhen. Beispielsweise können die zumindest erste Schicht und die zumindest zweite Schicht durch kovalente Bindungen, insbesondere mittels eines chemischen Vernetzungsmittels, miteinander verbunden sein. Weiterhin können die zumindest erste Schicht und die zumindest zweite Schicht miteinander verklebt sein. In einer vorteilhaften Variante kommt die Verbindung zwischen der zumindest ersten Schicht und der zumindest zweiten Schicht durch eine Lyophilisation, insbesondere Colyophilisation der Schichten, zustande.

Die zumindest erste Schicht besitzt bevorzugt eine dichte, insbesondere für Zellen undurchlässige, Struktur. Erfindungsgemäß kann es vorgesehen sein, dass die zumindest erste Schicht eine Struktur besitzt, die für Nährstoffe, biologische Wirkstoffe, medizinische bzw. pharmazeutische Wirkstoffe und/oder allgemein für niedermolekulare Verbindungen durchlässig ist. Beispielsweise kann die zumindest erste Schicht Poren mit einer Porengröße < 2 µm, insbesondere < 1 µm, aufweisen.

Die zumindest zweite Schicht weist bevorzugt eine poröse, insbesondere offenporöse, Struktur auf. Erfindungsgemäß ist es besonders bevorzugt, wenn die zumindest zweite Schicht eine für Zellen durchlässige bzw. infiltrierbare Struktur besitzt. Vorzugsweise weist die zumindest zweite Schicht Poren mit einer Porengröße zwischen 50 und 250 µm, insbesondere 130 und 200 µm, auf.

In einer weitergehenden Ausführungsform besitzt die zumindest erste Schicht eine membranartige Struktur. Insbsondere kann die zumindest erste Schicht als Membrankörper ausgebildet sein. Die zumindest zweite Schicht besitzt vorzugsweise eine schwammartige Struktur. Insbesondere kann die zumindest zweite Schicht als Schwammkörper ausgebildet sein. Bezüglich der Materialien, welche die zumindest erste Schicht und/oder die zumindest zweite Schicht aufweisen können, wird vollständig auf die bisher beschriebenen Trägermaterialien Bezug genommen. Erfindungsgemäß ist es jedoch bevorzugt, wenn die zumindest erste Schicht ein Material aufweist, welches ausgewählt ist aus der Gruppe bestehend aus Kollagen bzw. Salze davon, insbesondere Kollagen des Typs I und/oder III bzw. Salze davon, Elastin bzw. Salze davon, bioresorbierbare Polymere, Pericard, Komposite, Glykosaminoglykane bzw. Salze davon und Kombinationen davon. Die zumindest zweite Schicht weist vorzugsweise ein Material, insbesondere ein hydrophiles Material, auf, welches vorzugsweise ausgewählt ist aus der Gruppe bestehend aus Kollagen bzw. Salze davon, insbesondere Kollagen des Typs I und/oder III bzw. Salze davon, Hyaluronsäure bzw. Salze davon, Alginate bzw. Salze davon, Chitosan bzw. Salze davon, Gelatine bzw. Salze davon, prozessierte Materialien, Komposite, Fibrin bzw. Salze davon und Kombinationen davon. Das Material der zumindest zweiten Schicht kann außerdem vernetzt, insbesondere chemisch vernetzt, vorliegen.

In einer besonders bevorzugten Ausführungsform ist die zumindest erste Schicht eine Perikard-Membran, insbesondere eine Rinderperikardmembran, und die zumindest zweite Schicht ein Schwammkörper, vorzugsweise auf Basis von Kollagen des Typs I und/oder III bzw. von Salzen davon.

Die zumindest erste Schicht und die zumindest zweite Schicht weisen in einer weiteren Ausführungsform unterschiedliche Resorptionszeiten auf. Insbesondere besitzt die zumindest erste Schicht eine längere Resorptionszeit als die zumindest zweite Schicht. Bei den Resorptionszeiten handelt es sich vorzugsweise um in vivo gemessene Resorptionszeiten.

Bevorzugt handelt es sich bei dem Implantat um ein Knorpelimplantat, insbesondere um ein autologes Knorpelzellimplantat. Bevorzugt ist das Implantat ein Knorpelzellimplantat, das autologe Knorpelzellen und ein biphasisches dreidimensionales kollagen-basiertes Trägermaterial aufweist. Die erste Phase des Trägermaterials ist vorzugsweise als Schwammkörper, vorzugsweise mit säulenartig angeordneten und vorzugsweise untereinander in Verbindung stehenden Poren, ausgebildet. Dadurch ist mit besonderem Vorteil eine gleichmäßige dreidimensionale Verteilung der Knorpelzellen im Schwammkörper möglich. Die zweite Phase des Trägermaterials stellt vorzugsweise eine abdeckende und insbesondere reißfeste Membran, insbesondere Pericard-Membran, dar. Die Membran verhindert mit besonderem Vorteil in vivo ein Herauswandern der Knorpelzellen aus dem Defektbereich. Zudem ermöglicht die Membran eine einfache chirurgische Handhabbarkeit, wie beispielsweise ein sicheres und leichtes Einnähen des Implantats. Ein entsprechendes autologes Chondrozytenzellimplantat wird von der Anmelderin unter der Bezeichnung NOVO-CART 3D kommerziell vertrieben.

Das Implantat wird bevorzugt in der regenerativen Medizin, insbesondere auf dem Gebiet des Tissue Engineering, verwendet. Wie bereits erwähnt, eignet sich das Implantat vor allem zur Behandlung von Schäden und/oder Erkrankungen des menschlichen und/oder tierischen Stütz- und/oder Bewegungsapparates. Bei den Schäden kann es sich um Verletzungen handeln, welche insbesondere durch traumatische Ereignisse, beispielsweise Verkehrs- oder Sportunfälle, verursacht werden. Die Schäden, welche mittels des erfindungsgemäßen Implantats behandelt werden können, können aber auch die Folgen einer systemischen Erkrankung des menschlichen und/oder tierischen Stütz- und/oder Bewegungsapparates, beispielsweise einer Arthritis, sein. Bevorzugt wird das Implantat bei der autologen oder allogenen, besonders bevorzugt bei der autologen, Knorpelzelltransplantation verwendet. Ein weiteres Anwendungsgebiet des Implantats betrifft dessen Verwendung bei der Transplantation autologer und/oder allogener mesenchymaler Stammzellen, insbesondere für die Knorpel-, Sehnen-, Bänder- und/oder Knochenregeneration. Besonders bevorzugt ist die Verwendung des Implantats zur Behandlung von Schäden und/oder Erkrankungen von menschlichem und/oder tierischem Knorpelgewebe, insbesondere Gelenkknorpelgewebe und/oder Bandscheibengewebe. Eine weitere mögliche Verwendung des Implantats betrifft die Wundheilung am bzw. im menschlichen und/oder tierischen Körper.

Ein weiterer Aspekt der vorliegenden Erfindung betrifft eine therapeutische Zusammensetzung, umfassend Knorpelzellen und/oder Vorläuferzellen davon und einen knorpelspezifischen Kollagen-Typ. Die Knorpelzellen sind vorzugsweise aus der Gruppe bestehend aus Chondrozyten, Chondroblasten und Kombinationen davon ausgewählt. Bei den Vorläuferzellen handelt es sich in der Regel um Stammzellen, vorzugsweise mesenchymale Stammzellen. Die Knorpelzellen und/oder deren Vorläuferzellen können humanen und/oder tierischen Ursprungs sein. Bevorzugt sind die Knorpelzellen und/oder deren Vorläuferzellen humane Zellen. Des Weiteren können die Knorpelzellen und/oder deren Vorläuferzellen autologen oder allogenen Ursprungs sein. Bevorzugt handelt es sich bei den Knorpelzellen und/oder deren Vorläuferzellen um autologe Zellen. Der knorpelspezifische Kollagen-Typ stammt vorzugsweise aus einem biologischen, insbesondere menschlichen oder tierischen, Gewebe, vorzugsweise ausgewählt aus der Gruppe bestehend aus Augen-Hornhaut, Plazentagewebe, Aortagewebe, Synovialgewebe und Kombinationen davon. Bei dem korpelspezifischen Kollagen-Typ handelt es sich um Kollagen des Typs VI. Die therapeutische Zusammensetzung liegt in Form einer wässrigen Suspension vor. Die therapeutische Zusammensetzung eignet sich vor allem für eine in vitro-Kultivierung bzw. in vitro-Besiedelung eines Trägermaterials mit Knorpelzellen und/oder Vorläuferzellen davon. Weiterhin wird die Zusammensetzung vorzugsweise bei der autologen oder allogenen, vorzugsweise autologen, Knorpelzelltransplantation verwendet. Darüber hinaus kann die Zusammensetzung auch bei der Transplantation autologer und/oder allogener, vorzugsweise autologer, mesenchymaler Stammzellen, insbesondere für die Knorpel-, Sehnen-, Bänder- und/oder Knochenregeneration zum Einsatz kommen. Besonders bevorzugt ist die Verwendung der Zusammensetzung zur Behandlung von Schäden und/oder Erkrankungen von menschlichem und/oder tierischem Knorpelgewebe, insbesondere Gelenkknorpelgewebe und/oder Bandscheibengewebe. Bezüglich weiterer Merkmale und Vorteile der therapeutischen Zusammensetzung wird vollständig auf die bisherige Beschreibung Bezug genommen.

Ein weiterer Aspekt der vorliegenden Erfindung umfasst ein Herstellungsverfahren für das Implantat, umfassend die Schritte:
a) Bereitstellen von Knorpelzellen und/oder Vorläuferzellen davon und eines knorpelspezifischen Kollagen-Typs und
b) Beladen bzw. Beimpfen eines Trägermaterials mit den Knorpelzellen und/oder deren Vorläuferzellen und dem knorpelspezifischen Kollagen-Typ.

In einer bevorzugten Ausführungsform werden die Knorpelzellen und/ oder deren Vorläuferzellen zusammen mit dem knorpelspezifischen Kollagen-Typ in Form einer wässrigen Mischung bereitgestellt. Die wässrige Mischung wiederum kann in Form einer wässrigen Dispersion, wässrigen Suspension, wässrigen Lösung, eines Hydrogels oder einer wässrigen Paste bereitgestellt werden. Zur Herstellung der wässrigen Mischung wird bevorzugt eine wässrige Suspension der Knorpelzellen und/oder von deren Vorläuferzellen verwendet. Hierzu können die Knorpelzellen und/oder deren Vorläuferzellen gegebenenfalls enzymatisch vorbehandelt werden. Die Knorpelzellen selbst können entsprechenden Zelllinien, Zellkulturen und/oder Biopsieproben entnommen werden. Des Weiteren wird zur Herstellung der wässrigen Mischung bevorzugt eine Lösung oder Suspension des knorpelspezifischen Kollagen-Typs verwendet. Die Lösung bzw. Suspension weist vorzugsweise eine Konzentration des knorpelspezifischen Kollagen-Typs zwischen 10 µg/ml und 10 mg/ml auf. Zur Herstellung der wässrigen Mischung, insbesondere der Suspension, der Knorpelzellen und/oder von deren Vorläuferzellen und/oder der Lösung bzw. Suspension des knorpelspezifischen Kollagen-Typs, können grundsätzlich alle Biofluide verwendet werden. Beispielsweise können Biofluide aus der Gruppe bestehend aus Wasser, Pufferlösungen, Elektrolytlösungen, Nährstofflösungen und Körperflüssigkeiten, beispielsweise Blut und/oder Synovialflüssigkeit, verwendet werden. Die wässrige Mischung kann weiterhin biologische Wirkstoffe, insbesondere in einer Konzentration zwischen 10 und 500 ng/ml, enthalten.

In einer möglichen Ausführungsform wird das Trägermaterial zunächst lediglich mit den Knorpelzellen und/oder deren Vorläuferzellen beladen. In diesem Fall lässt man die Zellen normalerweise während eines bestimmten Zeitraumes, der typischerweise im Bereich von 1 bis 2 Tagen liegt, in das Trägermaterial hineinwandern, ehe das Trägermaterial anschließend mit dem knorpelspezifischen Kollagen-Typ beladen wird.

In einer bevorzugten Ausführungsform wird vor dem Beladen des Trägermaterials eine Co-Inkubation der Knorpelzellen und/oder von deren Vorläuferzellen und des knorpelspezifischen Kollagen-Typs, vorzugsweise in der bereits erwähnten wässrigen Mischung, durchgeführt. Die Co-Inkubation kann innerhalb eines Zeitraums zwischen 1 und 48 Stunden, insbesondere 2 und 36 Stunden, bevorzugt 4 und 24 Stunden, durchgeführt werden. Alternativ oder in Kombination dazu kann eine Co-Inkubation der Knorpelzellen und/oder von deren Vorläuferzellen und des knorpelspezifischen Kollagen-Typs auf bzw. in dem Trägermaterial durchgeführt werden. Die Inkubationszeit beträgt in diesem Fall bevorzugt zwischen 2 und 24 Stunden. Die in diesem Abschnitt beschriebenen Co-Inkubationen werden vorzugsweise in einem Temperaturbereich zwischen 0 und 39°C, insbesondere 30 und 39 °C, durchgeführt.

Die vorliegende Erfindung umfasst auch ein Implantat, welches nach einem der in den vorhergehenden Ausführungsformen beschriebenen Verfahren hergestellt bzw. herstellbar ist.

Ein weiterer Aspekt der Erfindung betrifft ein Herstellungsverfahren für die therapeutische Zusammensetzung. Erfindungsgemäß werden zur Herstellung der Zusammensetzung eine wässrige Flüssigkeit, vorzugsweise wässrige Suspension, enthaltend Knorpelzellen und/oder Vorläuferzellen davon, und eine wässrige Lösung (oder wässrige Suspension), enthaltend einen knorpelspezifischen Kollagen-Typ, gemischt.

Zur Herstellung der wässrigen Suspension können die Knorpelzellen und/oder deren Vorläuferzellen gegebenenfalls enzymatisch vorbehandelt werden. Die wässrige Lösung (oder wässrige Suspension) des knorpelspezifischen Kollagen-Typs weist vorzugsweise eine Konzentration des knorpelspezifischen Kollagen-Typs zwischen 10 µg/ml und 10 mg/ml auf. Bezüglich weiterer Merkmale und Eigenschaften, insbesondere in Bezug auf die Knorpelzellen und/oder deren Vorläuferzellen sowie den knorpelspezifischen Kollagen-Typ, wird vollständig auf die bisherige Beschreibung verwiesen.

Schließlich betrifft die vorliegende Erfindung auch die Verwendung eines Trägermaterials, von Knorpelzellen und/oder Vorläuferzellen davon und eines knorpelspezifischen Kollagen-Typs zur Herstellung eines Implantats, insbesondere zur Behandlung von Schäden und/oder Erkrankungen im Bereich des menschlichen und/oder tierischen Stütz- und/oder Bewegungsapparates. Des Weiteren betrifft die vorliegende Erfindung auch die Verwendung einer wässrigen Suspension, enthaltend Knorpelzellen und/oder Vorläuferzellen davon, und einer wässrigen Lösung (oder wässrigen Suspension), enthaltend einen knorpelspezifischen Kollagen-Typ, zur Herstellung einer therapeutischen Zusammensetzung, vorzugsweise zur Behandlung von Schäden und/oder Erkrankungen im Bereich des menschlichen und/oder tierischen Stütz- und/oder Bewegungsapparates. Bezüglich weiterer Merkmale und Einzelheiten, insbesondere in Bezug auf die Knorpelzellen und/oder deren Vorläuferzellen sowie den knorpelspezifischen Kollagen-Typ, wird ebenso vollständig auf die bisherige Beschreibung Bezug genommen.

Weitere Merkmale und Vorteile der Erfindung ergeben sich aus der nachfolgenden Beschreibung einer bevorzugten Ausführungsform in Form eines Beispiels in Verbindung mit den Unteransprüchen. Dabei können die Merkmale jeweils für sich alleine oder zu mehreren in Kombination miteinander verwirklicht sein. Das nachfolgende Bespiel dient lediglich der Erläuterung der vorliegenden Erfindung und ist in keiner Weise als einschränkend anzusehen.

### Ausführungsbeispiel

Es wurde Gelenkknorpel aus dem Radiuskopf einer 37-jährigen Patientin abgeschält, von allen Blut- und Geweberesten befreit und in einer Petrischale mit Skalpellen zerkleinert. Die Knorpelstücke wurden auf das Sieb einer Verdauungskammer mit Magnetröhre gegeben und mit 50 ml Hyaluronidase-Lösung (25 mg Hyaluronidase in 50 ml PBS (Phosphat gepufferte Kochsalzlösung bzw. phosphate buffered saline)) für 25 Minuten bei 37 °C gerührt. Danach wurde das Gewebe mit 50 ml 0.25 Gew.-% Trypsin/EDTA inkubiert (45 Minuten, 37 °C, gerührt) und 5 Minuten mit 50 ml DMEM (dulbecco's modifiziertes Eagle-Medium, high glucose) plus 10 Vol.-% FKS (fötales Kälberserum) gewaschen. Das Herauslösen der Knorpelzellen aus dem Gewebeverband erfolgte mit einer dreimaligen Kollagenase-Behandlung, bei der das Gewebe mit 50 ml Kollagenase-Lösung (25 mg in 50 ml DMEM plus 10 Vol.-% FKS plus 100 U/ml Penicillin plus 100 µg/ml Streptomycin) unter Rühren bei 37°C inkubiert und die Zellen aus der durchgetropften Lösung abzentrifugiert (500 x g, 5 Minuten) wurden. Das Gewebe wurde zuerst für 2 Stunden, später zweimal über Nacht mit Kollagenase verdaut. Anschließend wurden weitere Zellen durch Zugabe von DMEM plus 10 Vol.-% FKS aus dem Gewebe gespült und wie zuvor abzentrifugiert.

Die isolierten Knorpelzellen wurden anschließend in eine wässrige Suspension gebracht. Die wässrige Suspension wurde anschließend mit einer wässrigen Lösung, enthaltend den Kollagen-Typ VI, gemischt. In der hergestellten Mischung wurden die Knorpelzellen und das Kollagen vom Typ VI während eines Zeitraums von etwa 18 Stunden co-inkubiert. Anschließend wurde ein bioverträgliches Trägermaterial (scaffold) mit der Mischung beladen. Bei dem Trägermaterial handelte es sich um ein Kompositmaterial auf der Basis einer membranartig ausgebildeten ersten Schicht und einer schwammartig ausgebildeten zweiten Schicht. Die erste Schicht stellte eine Pericard-Membran dar. Die zweite Schicht wurde im Wesentlichen von vernetzter Gelatine gebildet. Nach Beladung des Kompositmaterials ließ man etwa 24 Stunden verstreichen, damit die schwammartig ausgebildete zweite Schicht in ausreichendem Maße von den Knorpelzellen infiltriert werden konnte.

Anschließend wurde das so beladene Kompositmaterial erfolgreich in eine defekte Gelenkknorpelregion der oben erwähnten Patientin implantiert.

## Patentansprüche

1. Implantat, umfassend ein Trägermaterial, Knorpelzellen und/oder Vorläuferzellen davon und einen knorpelspezifischen Kollagen-Typ, insbesondere zur Verwendung in der Behandlung von Schäden und/oder Erkrankungen im Bereich des menschlichen und/oder tierischen Stütz- und/oder Bewegungsapparates, **dadurch gekennzeichnet, dass** es sich bei dem knorpelspezifischen Kollagen-Typ um Kollagen des Typs VI handelt.

2. Implantat nach Anspruch 1, **dadurch gekennzeichnet, dass** die Knorpelzellen und/oder deren Vorläuferzellen autologen oder allogenen, vorzugsweise autologen, Ursprungs sind.

3. Implantat nach Anspruch 1 oder 2, **dadurch gekennzeichnet, dass** es sich bei den Knorpelzellen um primäre Knorpelzellen handelt.

4. Implantat nach einem der vorhergehenden Ansprüche, **dadurch gekennzeichnet, dass** die Knorpelzellen und/oder deren Vorläuferzellen durch eine in vitro-Besiedelung oder in vitro-Kultivierung in das Implantat eingelagert sind.

5. Implantat nach einem der vorhergehenden Ansprüche, **dadurch gekennzeichnet, dass** der knorpelspezifische Kollagen-Typ tierischen, insbesondere bovinen, porcinen und/oder equinen, und/ oder humanen Ursprungs ist.

6. Implantat nach einem der vorhergehenden Ansprüche, **dadurch gekennzeichnet, dass** der knorpelspezifische Kollagen-Typ aus einem Gewebe aus der Gruppe bestehend aus Augen-Hornhaut, Plazentagewebe, Aortagewebe und Synovialgewebe stammt.

7. Implantat nach einem der vorhergehenden Ansprüche, **dadurch gekennzeichnet, dass** das Implantat Chondrone und/oder chondronartige Vorstufen aufweist.

8. Implantat nach einem der vorhergehenden Ansprüche, **dadurch gekennzeichnet, dass** das Trägermaterial ein Material, ausgewählt aus der Gruppe bestehend aus Proteine bzw. Salze davon, Polysaccharide bzw. Salze davon, Polyhydroxyalkanoate, tierische Membranen und Kombinationen davon, umfasst.

9. Implantat nach einem der vorhergehenden Ansprüche, **dadurch gekennzeichnet, dass** das Trägermaterial mehrschichtig aufgebaut ist, vorzugsweise zumindest eine erste Schicht und zumindest eine zweite Schicht aufweist, wobei vorzugsweise die zumindest erste Schicht eine membranartige Struktur und die zumindest zweite Schicht eine schwammartige Struktur besitzt.

10. Implantat nach einem der vorhergehenden Ansprüche zur Verwendung in der regenerativen Medizin, insbesondere zur Verwendung auf dem Gebiet des Tissue Engineering, in der Wundheilung, bei der autologen oder allogenen Knorpelzelltransplantation, bei der Transplantation autologer oder allogener mesenchymaler Stammzellen für die Knorpel-, Sehnen-, Bänder- und/oder Knochenregeneration und/oder zur Behandlung von Schäden und/oder Erkrankungen von menschlichem und/oder tierischem Knorpelgewebe.

11. Therapeutische Zusammensetzung, umfassend Knorpelzellen und/oder Vorläuferzellen davon und einen knorpelspezifischen Kollagen-Typ, vorzugsweise zur Verwendung in der Behandlung von Schäden und/oder Erkrankungen im Bereich des menschlichen und/oder tierischen Stütz- und/oder Bewegungsapparates, **dadurch gekennzeichnet, dass** es sich bei dem knorpelspezifischen Kollagen-Typ um Kollagen des Typs VI handelt und die therapeutische Zusammensetzung in Form einer wässrigen Suspension vorliegt.

12. Verfahren zur Herstellung eines Implantats nach einem der Ansprüche 1 bis 10, umfassend die Schritte:
a) Bereitstellen der Knorpelzellen und/oder Vorläuferzellen davon und des knorpelspezifischen Kollagen Typs und
b) Beladen des Trägermaterials mit den Knorpelzellen und/oder deren Vorläuferzellen und dem knorpelspezifischen Kollagen-Typ.

13. Verfahren zur Herstellung einer therapeutischen Zusammensetzung nach Anspruch 11, wobei eine wässrige Suspension, enthaltend Knorpelzellen und/oder Vorläuferzellen davon, und eine wässrige Lösung, enthaltend einen knorpelspezifischen Kollagen-Typ, gemischt werden, **dadurch gekennzeichnet, dass** es sich bei dem knorpelspezifischen Kollagen um Kollagen des Typs VI handelt.

14. Verwendung einer wässrigen Suspension, enthaltend Knorpelzellen und/oder Vorläuferzellen davon, und einer wässrigen Lösung, enthaltend einen knorpelspezifischen Kollagen-Typ, zur Herstellung einer therapeutischen Zusammensetzung, insbesondere zur Verwendung in der Behandlung von Schäden und/oder Erkrankungen im Bereich des menschlichen und/oder tierischen Stütz- und/oder Bewegungsapparates, **dadurch gekennzeichnet, dass** es sich bei dem knorpelspezifischen Kollagen um Kollagen des Typs VI handelt.

15. Verwendung eines Trägermaterials, von Knorpelzellen und/oder Vorläuferzellen davon und eines knorpelspezifischen Kollagen-Typs zur Herstellung eines Implantats nach einem der Ansprüche 1 bis 10, **dadurch gekennzeichnet, dass** es sich bei dem knorpelspezifischen Kollagen um Kollagen des Typs VI handelt.

## Claims

1. An implant comprising a support material, cartilage cells and/or precursor cells thereof, and a cartilage-specific collagen type, in particular for use in treating damage and/or diseases relating to the human and/or animal musculoskeletal and/or locomotor system, **characterized in that** the cartilage-specific collagen type is type VI collagen.

2. The implant according to claim 1, **characterized in that** the cartilage cells and/or precursor cells thereof are of autologous or allogeneic, preferably autologous, origin.

3. The implant according to claim 1 or 2, **characterized in that** the cartilage cells are primary cartilage cells.

4. The implant according to any one of the preceding claims, **characterized in that** the cartilage cells and/or precursor cells thereof are embedded in the implant by means of in vitro colonization or in vitro culturing.

5. The implant according to any one of the preceding claims, **characterized in that** the cartilage-specific collagen type is of animal, more particularly bovine, porcine and/or equine, and/or human origin.

6. The implant according to any one of the preceding claims, **characterized in that** the cartilage-specific collagen type originates from a tissue from the group consisting of cornea, placental tissue, aortic tissue, and synovial tissue.

7. The implant according to any one of the preceding claims, **characterized in that** the implant has chondrons and/or chondron-like precursors.

8. The implant according to any one of the preceding claims, **characterized in that** the support material comprises a material selected from the group consisting of proteins and salts thereof, polysaccharides and salts thereof, polyhydroxyalkanoates, animal membranes, and combinations thereof.

9. The implant according to any one of the preceding claims, **characterized in that** the support material is constructed as a multilayer, preferably has at least a first layer and at least a second layer, wherein preferably the at least first layer has a membrane-like structure and the at least second layer has a sponge-like structure.

10. The implant according to any one of the preceding claims for use in regenerative medicine, in particular for use in the field of tissue engineering, in wound healing, in autologous or allogeneic cartilage cell transplantation, in the transplantation of autologous or allogeneic mesenchymal stem cells for cartilage, tendon, ligament and/or bone regeneration, and/or for treating damage to and/or diseases of human and/or animal cartilage tissue.

11. A therapeutic composition, comprising cartilage cells and/or precursor cells thereof and a cartilage-specific collagen type, preferably for use in treating damage and/or diseases relating to the human and/or animal musculoskeletal and/or locomotor system, **characterized in that** the cartilage-specific collagen type is type VI collagen and the therapeutic composition is provided in the form of an aqueous suspension.

12. A method for preparing an implant according to any one of the claims 1 to 10, comprising the steps:
a) providing cartilage cells and/or precursor cells thereof and a cartilage-specific collagen type, and
b) loading a support material with said cartilage cells and/or precursor cells thereof and said cartilage-specific collagen type.

13. A method for preparing a therapeutic composition according to claim 11, wherein an aqueous suspension, containing cartilage cells and/or precursor cells thereof, and an aqueous solution, containing a cartilage-specific collagen type, are mixed, **characterized in that** the cartilage-specific collagen type is type VI collagen.

14. The use of an aqueous suspension, containing cartilage cells and/or precursor cells thereof, and of an aqueous solution, containing a cartilage-specific collagen type, for preparing a therapeutic composition, in particular for use in treating damage and/or diseases relating to the human and/or animal musculoskeletal and/or locomotor system, **characterized in that** the cartilage-specific collagen type is type VI collagen.

15. The use of a support material, of cartilage cells and/or precursor cells thereof, and of a cartilage-specific collagen type to prepare an implant according to any one of the claims 1 to 10, **characterized in that** the cartilage-specific collagen type is type VI collagen.

## Revendications

1. Implant, comprenant un matériau support, des chondrocytes et/ou des cellules précurseurs de ces derniers, et un type de collagène spécifique du cartilage, en particulier pour une utilisation dans le traitement de lésions et/ou de maladies dans le domaine de l'appareil de soutien et/ou de l'appareil locomoteur humain et/ou animal, **caractérisé en ce que**, pour ce qui concerne le type de collagène spécifique du cartilage, il s'agit du collagène de type VI.

2. Implant selon la revendication 1, **caractérisé en ce que** les chondrocytes et/ou leurs cellules précurseurs sont d'origine autologue ou allogène, de préférence autologue.

3. Implant selon la revendication 1 ou 2, **caractérisé en ce que**, pour ce qui concerne les chondrocytes, il s'agit de chondrocytes primaires.

4. Implant selon l'une des revendications précédentes, **caractérisé en ce que** les chondrocytes et/ou leurs cellules précurseurs sont incorporés dans l'implant par une colonisation in vitro ou par une culture in vitro.

5. Implant selon l'une des revendications précédentes, **caractérisé en ce que** le type de collagène spécifique du cartilage est d'origine animale, en particulier bovine, porcine et/ou équine et/ou humaine.

6. Implant selon l'une des revendications précédentes, **caractérisé en ce que** le type de collagène spécifique du cartilage provient d'un tissu du groupe consistant en la cornée de l'oeil, le tissu placentaire, le tissu aortique et le tissu synovial.

7. Implant selon l'une des revendications précédentes, **caractérisé en ce que** l'implant comprend des chondrones et/ou des précurseurs de type chondrone.

8. Implant selon l'une des revendications précédentes, **caractérisé en ce que** le matériau support comprend un matériau choisi dans le groupe consistant en les protéines ou les sels de celles-ci, les polysaccharides ou les sels de ceux-ci, les polyhydroxyalcanoates, les membranes animales et les combinaisons de ceux-ci.

9. Implant selon l'une des revendications précédentes, **caractérisé en ce que** le matériau support a une structure multicouche, et comprend de préférence au moins une première couche et au moins une deuxième couche, de préférence la ou les premières couches ayant une structure de type membrane et la ou les deuxièmes couches ayant une structure de type éponge.

10. Implant selon l'une des revendications précédentes pour une utilisation en médecine régénérative en particulier pour une utilisation dans le domaine de l'ingénierie tissulaire, dans la cicatrisation, dans la transplantation autologue ou allogène de chondrocytes, lors de la transplantation de cellules souches mésenchymateuses autologues ou allogènes pour la régénération du cartilage, des tendons, des ligaments et/ou des os, et/ou pour le traitement de lésions et/ou de maladies du tissu cartilagineux humain et/ou animal.

11. Composition thérapeutique comprenant des chondrocytes et/ou des cellules précurseurs de celles-ci, et un type de collagène spécifique du cartilage, de préférence pour une utilisation dans le traitement de lésions et/ou de maladies dans le domaine de l'appareil de soutien et/ou de l'appareil locomoteur humain et/ou animal, **caractérisée en ce que**, pour ce qui concerne le type de collagène spécifique du cartilage, il s'agit du collagène de type VI, et la composition thérapeutique se présente sous forme d'une suspension aqueuse.

12. Procédé de fabrication d'un implant selon l'une des revendications 1 à 10, comprenant les étapes :
a) mise à disposition des chondrocytes et/ou des précurseurs de ceux-ci, et du type de collagène spécifique du cartilage, et
b) chargement du matériau support avec les chondrocytes et/ou leurs cellules précurseurs, et le type de collagène spécifique du cartilage.

13. Procédé de préparation d'une composition thérapeutique selon la revendication 11, dans lequel on mélange une suspension aqueuse contenant des chondrocytes et/ou des cellules précurseurs de ceux-ci, et une solution aqueuse contenant un type de collagène spécifique du cartilage, **caractérisé en ce que**, pour ce qui concerne le collagène spécifique du cartilage, il s'agit du collagène de type VI.

14. Utilisation d'une suspension aqueuse contenant des chondrocytes et/ou des cellules précurseurs de ceux-ci, et d'une solution aqueuse contenant un type de collagène spécifique du cartilage, pour la préparation d'une composition thérapeutique, en particulier pour une utilisation dans le traitement de lésions et/ou de maladies dans le domaine de l'appareil de soutien et/ou de l'appareil locomoteur humain et/ou animal, **caractérisée en ce que**, pour ce qui concerne le collagène spécifique du cartilage, il s'agit du collagène de type VI.

15. Utilisation d'un matériau support, de chondrocytes et/ou de cellules précurseurs de ceux-ci et d'un type de collagène spécifique du cartilage pour la fabrication d'un implant selon l'une des revendications 1 à 10, **caractérisée en ce que**, pour ce qui concerne le collagène spécifique du cartilage, il s'agit du collagène de type VI.
